# EUROPÄISCHE PATENTSCHRIFT

(11) **EP 2 505 707 B1**
(45) Veröffentlichungstag und Bekanntmachung des Hinweises auf die Patenterteilung: **31.07.2013**
(21) Anmeldenummer: 11002732.3
(22) Anmeldetag: 01.04.2011
(51) Int. Cl.: D04H 3/11, A61F 13/62, A44B 18/00, A61F 13/514

(54) **Die Verwendung von wasserstrahlverfestigten Vliesen als Klettverschlusskomponente**
The use of hydroentangled non-woven fabrics as hook-and-loop component
L`utilisation des non-tissés renforcés par jet d'eau en tant que composant de joint auto-agrippant

(43) Veröffentlichungstag der Anmeldung: 03.10.2012
(73) Patentinhaber: RKW SE, 67227 Frankenthal (DE)
(72) Erfinder: Kirsch, Andreas, 31167 Bockenem (DE); Börmann, Ludwig, 83547 Babensham (DE); Schreiner, Günter, 83530 Schnaitsee (DE)
(74) Vertreter: Wagner, Jutta

(56) Entgegenhaltungen:
- US-A- 4 329 763
- US-A- 5 023 130
- US-A1- 2009 068 394
- US-B1- 6 321 425

## Beschreibung

Die vorliegende Erfindung betrifft die Verwendung von wasserstrahlverfestigten Vliesstoffen aus Endlosfilamenten als Schlaufenkomponente von Klettverschlüssen sowie Schlaufenkomponenten für Klettverschlüsse aus wasserstrahlverfestigten Vliesstoffen aus Endlosfilamenten.

Bei Windeln werden heute unterschiedliche Varianten von Verschlusssystemen benutzt, wobei das Verschließen der Windel entweder mit einem klebenden Tape auf einer Folie oder mit einem Häkchentape (hook) auf eine Schlaufenlage (loop) erfolgt. Traditionelle Haken/Schlaufenvarianten, im allgemeinen als Klettverschluss bezeichnet, sind die hochwertigeren Verschlusssysteme, bei denen die Schlaufenlage überwiegend aus textilen Materialien bestehen. Sie haben im Empfinden des Konsumenten die höherwertige Einschätzung, da diese als Verschlußsysteme auch von Bekleidung und Schuhen bekannt sind. Bei kostensensitiven Produkten für den temporären oder einmaligen Gebrauch, müssen preiswertere Hakenschlaufenvarianten eingesetzt werden, die zu geringeren Kostenstrukturen hergestellt werden können. Im Bereich von absorbierenden Einwegartikeln, insbesondere Windeln, werden solche Klettverschlussvorrichtungen z.B. in WO 96/22065, EP 719 533, EP 721 770 und WO 95/25496 beschrieben.

Die Verwendung bestehender Klettverschlüsse an Produkten zum temporären oder einmaligen Gebrauch ist aufgrund der Kosten der Befestiger, relativ zu anderen Befestigertypen, beschränkt. Für diese Produktgruppe, speziell für absorbierende Einwegprodukte, wie Windeln ist damit die Forderung nach kostengünstigen Lösungsalternativen von hoher Priorität.

Aus diesem Grund wurde schon früh versucht, Schlaufensysteme auf Basis von Vliesstoffen zu entwickeln. Die mit den zunächst üblichen Prozessen hergestellten Vliesstoffe mussten nachbehandelt werden, um das Fasergelege zu einer schlaufenbildenden Fasermatte zu formen (z. B. nachträgliche spezielle thermische Bondierung).

Zwar werden die Außenlagen von absorbierenden Einwegartikeln, insbesondere von Windeln, vorzugsweise mit einer Oberfläche hergestellt, die einem Textilgewebe in Aussehen und dem Gefühl bei der Berührung (touch) möglichst ähnlich ist. Dazu wird als Außenlage zumeist ein Verbund aus flüssigkeitsdichter Folie und einem Vlies als textil-ähnlicher äußerer Schicht vorgesehen. Trotz der Erfolge in dieser Hinsicht reichen aber die derzeitigen Oberflächen zumeist nicht aus, um die männliche Hakenkomponente einer Klettverschlussvorrichtung in dieser Oberfläche direkt zu verhaken. Die hierbei erzielten Haftkräfte sind zu gering.

Eine Ausbildung der gesamten äußeren Oberfläche mit dem hierzu erforderlichen Volumen des Vlieses verbietet sich aus Kostengründen, da dies nur über Vliese mit höherer Dicke bzw. höherem Flächengewicht des Vlieses möglich ist. So kann beispielsweise ein Vlies durch entsprechende Auffaltung eine ausreichende Haftung bieten. Aus Kostengründen wird aber ein stetig verminderter Materialaufwand gefordert und die immer weiter reduzierten Materialstärken, typisch sind derzeit etwa 15 bis 30 g/m², erlauben es auch bei einer Vordehnung mittels Ring-Rolling nicht, eine als Schlaufenkomponente geeignete Oberfläche zu adäquaten Kosten anzubieten. Im Vergleich zu entsprechend voluminöseren Vliesen aus den gängigen Polyolefinmaterialien sind die derzeit üblichen Polyestergewebe oder -gewirke als separat hergestellte und auf das Backsheet aufgebrachte Schlaufenkomponente trotz des wesentlich höheren Materialpreises der Polyestervlies und dem zusätzlich notwendigen Befestigungsschritt preisgünstiger.

In WO 98/11855 wird als Lösung eine Schlaufenkomponente vorgeschlagen, die einstückig mit der Außenlage (Backsheet) eines Einwegartikels ausgebildet ist. Dazu soll das Backsheet aus je einer inneren und einer äußeren Lage bestehen, die miteinander verbunden sind, wobei die äußere Lage aus Vlies im landing zone-Bereich/Schlaufenbereich zwingend durch eine Vordehnung ausreichend voluminös gemacht wird und in dem Bereich, der später die Schlaufenkomponente der Klettverschlussvorrichtung bildet, nicht oder weniger mit der inneren Lage verbunden ist. Auch bei diesem Verfahren muss jedoch für eine ausreichende Schließkraft der Bereich der landing zone besonders mit Hilfe der Vordehnung hergestellt werden.

EP 1 915 918 schlägt ein Verfahren zur Herstellung von Verbunden, welche sich als Schlaufenkomponente von Klettverschlüssen eignen, vor, bei dem eine Folie und ein Vlies auf einem Heizzylinder zusammengeführt werden, wobei die Folie von dem Heizylinder bis zum schmelzeflüssigen Zustand erhitzt wird. Dabei bildet sich ein Verbund von Folie und Vlies, den man um 1 bis 10 % in Maschinenrichtung schrumpfen lässt, so dass das Vlies durch die Verminderung seiner Dimension in der Fläche an Höhe zunimmt. Anschließend wird der Verbund in einem gekühlten Walzenspalt auf eine Temperatur unterhalb der Schmelztemperatur der Folie gekühlt. Solche Verbunde sind als Backsheet geeignet und erlauben es, insbesondere speziell angepasste Haken an dem Vlies zu verankern. Dieses Verfahren setzt voraus, dass die Folie in der Hitze schrumpft und erlaubt es nicht, das isolierte Vlies als Schlaufenkomponente an einem Artikel anzubringen.

In US 2009/0068394 A1 wird ein wasserstrahlverfestigtes Vlies als Schlaufenkomponente beschrieben, das zwingend 3 bis 80 Öffnungen pro m² aufweist, bei denen die ineinander verhakten Fasern an den seitlichen Wandungen der Öffnungen intakt sind. Das Vlies erlaubt nur eine nicht zu hohe Anzahl von Öffnungs- und Schließvorgängen, da es eine verhältnismäßig hohe Faserauflösung schon bei geringen Öffnungszyklen aufweist. Es soll sich auch zur Verwendung als Backsheet eignen. Die Flächengewichte in den Beispielen sind mit 33 g/m² aber noch recht hoch und so schlägt auch die US 2009/0068394 A1 vor, nachträglich fixierte Streifen des Vlieses als Schlaufenkomponente einzusetzen. Das Einbringen der Öffnungen ist zudem aufwändig.

Es besteht daher weiterhin die Aufgabe, die Herstellung von Klettverschlussvorrichtungen bei absorbierenden Einwegartikeln zu vereinfachen bzw. kostengünstiger zu ermöglichen.

Überraschend wurde nun gefunden, dass wasserstrahlverfestigte Vliesstoffe aus Endlosfilamenten selbst ohne eine Nachbehandlung oder die Verbindung mit einer weiteren Materiallage den notwendigen schlaufenartigen Faserflor aufweisen, in welchem die Hakenkomponente von Klettverschlüssen verhakt werden kann.

Die vorliegende Erfindung löst daher die oben genannten Probleme der bekannten Materialien, durch Verwendung eines wasserstrahlverfestigten Vliesstoffes aus Endlosfilamenten als Schlaufenkomponente von Klettverschlüssen gemäß Anspruch 1.

Erfindungsgemäß kommen wasserstrahlverfestigte Endlosfaservliese zum Einsatz. Die wasserstrahlverfestigten Vliesstoffe aus Endlosfilamenten bieten aufgrund ihres Herstellungsprozesses einen Faserflor, der schlaufenartig ausgebildet ist und damit den Haken hinreichend Möglichkeit zur Verankerung bietet.

Gut geeignet sind Vliesstoffe mit einem Flächengewicht von 10 g/m² bis 50 g/m², vorzugsweise von 15 bis 30 g/m². In Einzelfällen kann das Flächengewicht bis 150 g/m² betragen. Die Faserstruktur, d.h. die Offenheit des Vliesstoffes sowie der Verschlingungsgrad der einzelnen Fasern kann gezielt durch die Prozessparameter der Wasserstrahlverfestigung beeinflusst und gesteuert werden, um die durch die Wasserstrahlen generierten Schlaufen größer oder kleiner, fester oder lockerer zu gestalten. Dies geschieht durch Variationen des Wasserdrucks, der Wassermenge und/oder der Anzahl und Anordnung der Wasserstrahlen. Es ist auch möglich, mittels Wasserstrahlen einen Vliesstoff mit einer Schlaufen in Schlaufen Struktur, ähnlich einer Strickware, zu erzeugen. Wasserstrahlverfestigte Vliesstoffe aus Endlofilamenten sind beispielsweise unter der Bezeichnung HyJet® von der RKW SE kommerziell erhältlich.

Es ist von Vorteil, dass wasserstrahlverfestigte Vliesstoffe aus Endlosfilamenten erfindungsgemäß ohne weitere Behandlungen als Schlaufenkomponente verwendet werden können. Insbesondere ist es nicht notwendig, definierte Öffnungen einzubringen und erfolgt auch nicht. Die in der Struktur des Vliesstoffes vorhandenen unregelmäßig verteilten Öffnungsstrukturen sind von den nach dem Stand der Technik eingebrachten Öffnungen zu unterscheiden und werden nicht als Öffnungen angesehen.

Es ist weiterhin vorteilhaft, dass auch keine Bindung nötig ist, weder durch Einbringen von Bondierungspunkten noch durch Vorsehen gebundener Bereiche mit nicht gebundenen Punkten/Flächen.

Je nach Art der verwendeten Hakenvarianten (hooks) kann der Freiheitsgrad der gebildeten Schlaufen aber zusätzlich beeinflusst bzw. zielgerichtet definiert werden, indem der Faserflor durch thermische Verfestigung und/oder Ultraschallschweißung und/oder Verklebung an diskreten Stellen gebunden wird. Hier reicht eine Bindung der Fasern in einer Fläche von 1-25 %, vorzugsweise 2 bis 20 % der gesamten Fläche. Diese Bindung kann nach der Wasserstrahlverfestigung erfolgen. Bevorzugt wird eine thermische Verfestigung, insbesondere mittels eines Thermobondkalanders. Durch dessen Stellparameter kann der Grad der Verfestigung zielgerichtet eingestellt werden. Die Bondierung kann dabei punktuell mit verschiedenen Geometrien wie Kreis, Rind, Raute, oder in Linienform erfolgen.

Der Vliesstoff kann als solches bei der Herstellung von Windeln oder anderen Produkten an die vorgesehene Stelle, z.B. an der Außenhaut der Windel, platziert werden und bietet damit die als Landing Zone bezeichnete Zielfläche für die Hakentapes. Die Platzierung erfolgt hierbei durch eine Fixierung, vorzugsweise Verklebung, auf die z.B. Windelaußenhaut, wodurch die Endlosfilamente an der Unterseite des Vlieses an der Außenhaut fixiert werden. Durch diese Verklebung wird der Freiheitsgrad der einzelnen Schlaufen begrenzt und an die Erfordernisse des Hakenmaterials angepasst.

Eine Ausführungsvariante besteht darin, den erfindungsgemäßen Vliesstoff mit einer weiteren Lage, vorzugsweise einer Polymerfolie zu verbinden, um damit einen vorgefertigten Vlies-Folienverbund als Backsheet oder Landing Zone-Material zu realisieren. Hierfür stehen unterschiedliche Prozesse zur Verfügung: Direktextrusion (z.B. mit Vakuum oder im Walzenspalt), Düsenbeschichtung, Kleberlaminierung, welche alle brauchbar sind.

Durch Variationen der Prozessparameter kann bei der Direktextrusion die Eindringtiefe des Schlaufenmaterials in die Folienoberfläche gesteuert werden, so dass die zur Verfügung stehende Schlaufengröße und -länge entsprechend der verwendeten Hakenmaterialien zielgerichtet modifiziert werden kann. Prinzipiell sind alle Methoden zur Aufbringung von Polymeren (oder EVA, Hotmelt etc.) geeignet, um eine definierte Einbindung des Schlaufenmaterials zu erzielen, wobei der Polymerauftrag sowohl vollflächig als auch in definierten Teilflächen erfolgen kann. Durch teilflächigen Auftrag kann die Schlaufengröße und -länge ebenfalls gesteuert werden.

Besonders bevorzugt ist ein Verbund von wasserstrahlverfestigten Vliesstoffen aus Endlosfilamenten mit einer Folie, der mittels dem aus WO 2006/024394 bekannten Thermo-Laminierverfahren hergestellt ist. Damit ist die Herstellung einer kostengünstigen Landing Zone-Variante bei gleichzeitiger Steuerung der Schlaufenbildung besonders leicht und reproduzierbar möglich. Erfindungsgemäß kann hier die Eindringtiefe der Vliesstoffschlaufen in die Folienschicht exakt definiert werden und damit die Schlaufengeometrie in idealer Weise auf die unterschiedlichen Hakengeometrien angepasst werden. Entgegen EP 1 915 918 wird erfindungsgemäß jedoch ein Schrumpfen des Verbundes vermieden, der Vliesstoff soll seine Flächenausdehnung im wesentlichen nicht verändern. Ein geringer Schrumpf von z.B. bis zu 1 %, bevorzugt bis zu 0,5 % stört aber nicht.

Die Eindringtiefe des Folienmaterials bzw. Klebstoffes in den Vliesstoff kann zwischen 1 und 50% des Vliesstoffes betragen, vorzugsweise zwischen 10 und 40 %. Dabei werden gute Fixierungen der Schlaufen erzielt, ohne dass der Vliesstoff seine textilartige, weiche Oberfläche verliert. Die Eindringtiefe sollte wenigstens 1 %, vorzugsweise wenigstens 2 % der Dicke des Vliesstoffes betragen, damit die Schlaufen beim Lösen der Hakenkomponente nicht zu weit aus dem Vlies gelöst werden.

Als Material für die Herstellung der Endlosfilamente für die Vliesstoffe eignen sich alle spinnbaren Polymere, wie beispielsweise Polyester, PLA, Polyolefine, insbesondere Polypropylen und Polyethylen. Generell können auch andere Polymere, welche ein Spinnen zu Fasern erlauben, verwendet werden. Besonders bevorzugt bestehen die Endlosfilamente und damit der Vliesstoff aus Polypropylenmaterial und/oder Polyethylenmaterial oder einem biologisch abbaubaren Material. Dabei enthält das Material Polypropylen und/oder Polyethylen oder ein biologisch abbaubares Material und die üblichen Additive, wie z.B. Verarbeitungshilfsmittel, sowie etwaige herstellungsbedingte Bestandteile.

Die Filamente sollten eine Stärke zwischen 10 und 70 µm aufweisen, um auf der einen Seite einen textilen Charakter zu gewährleisten, auf der anderen Seite eine genügende Filamentstabilität zu garantieren.

Mit den erfindungsgemäßen Vliesstoffen bzw. Verbunden werden mit üblichen Haken, z.B. der Fa. Binder, Velcro, 3M, die geforderten Werte der Hakenscherkräfte von > 10 N/2,54 cm erfüllt. Typischerweise werden Scherfestigkeiten von 10 bis 100 N/2,54 cm, vorzugsweise von 20 bis 80 N/2,54 cm, besonders bevorzugt von 30 bis 70 N/2,54 cm erreicht.Beim Öffnen wird eine maximale Peelkraft von 30 N/2,54 cm nicht überschritten. Vorzugsweise liegt die Peelkraft im Bereich von 1 bis 25 N/2,54 cm, besonders bevorzugt von 3 bis 15 N/2,54 cm. Auch bei mehrmaligem Öffnen und Schließen, , werden die Fasern nicht so weit aus dem Vliesstoff herausgerissen, dass die Hakenkräfte unter die gewünschten Werte sinken oder der Vliesstoff zerrupft aussieht.

Die Erfindung soll anhand der folgenden Figuren erläutert werden, ohne jedoch auf die speziell beschriebenen Ausführungsformen beschränkt zu sein. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

Figur 1 zeigt einen erfindungsgemäßen Vliesstoff.

Figur 2 zeigt einen erfindungsgemäßen Vliesstoff mit diskreten Bondierpunkten.

Figur 3 zeigt einen Verbund aus Vliesstoff und Folie.

Figur 4 zeigt einen weiteren Verbund aus Vliesstoff und Folie.

Figur 5 zeigt eine Windel.

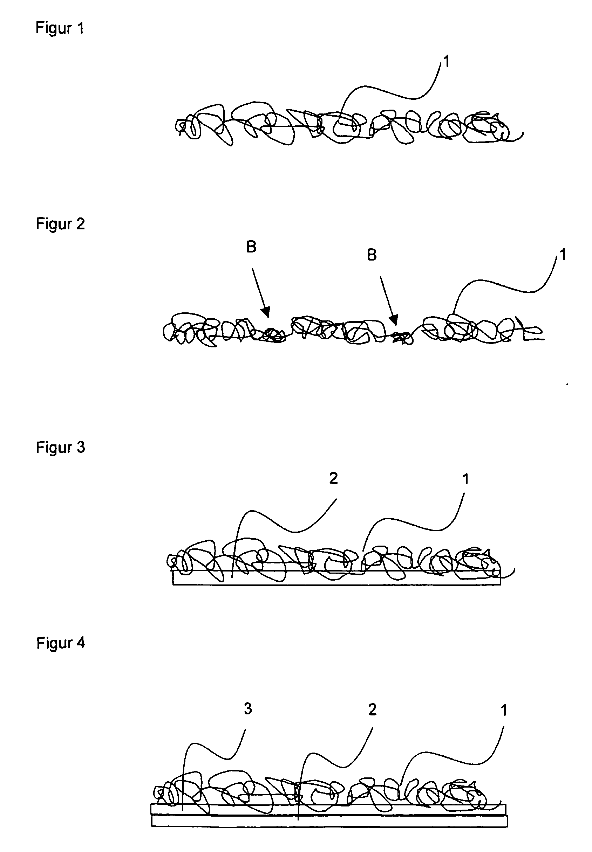

In Figur 1 ist schematisch ein Ausschnitt aus einem erfindungsgemäßen Vliesstoff schematisch dargestellt. Der Vliesstoff 1 hat weder Bondierpunkte noch Öffnungen oder andere Inhomogenitäten. Die durch das Wasserstrahlverfestigen erreichte Struktur bildet allein schon ausreichend voluminöse Schlaufen, so dass die üblichen Haken die notwendige Haftung erreichen. Ein solcher Vliesstoff kann als Landing-zone auf eine Windel oder ein anderes Hygieneprodukt oder auf Wegwerfbekleidung auflaminiert werden. Durch die Befestigung der Filamente auf der Unterlage wird die Schlaufenstruktur fixiert und das Herauslösen der Endlosfilamente limitiert.

Figur 2 zeigt eine Variante bei der mittels diskreter Bondierung der Fasern an den Bondierungspunkten B, die eine Fläche von 1 -25 % der Gesamtfläche einnehmen, die Schlaufenstruktur zielgerichtet fixiert wird. Dieses Bondieren kann thermisch, durch Ultraschall oder chemisch mit Klebstoffen erfolgen. Die Form der Bondierungspunkte B kann in an sich bekannter Weise gewählt werden, geeignet sind z.B. Kreis, Raute, Quadrat, etc. jeweils gefüllt oder ungefüllt, oder in Linienform.

Figur 3 zeigt eine Variante, bei welcher der Vliesstoff 1 mit einer Folie 2 laminiert ist. Zur Laminierung eignet sich beispielsweise das Direktextrusionsverfahren oder auch das in WO 2006/024394 beschriebene Thermolaminierverfahren. Dabei dringt das Material der Folie 2 in den Vliesstoff 1 ein. Als brauchbar haben sich Eindringtiefen von 1-50 %der Vliesstoffdicke erwiesen.

In einer vierten, in Figur 4 gezeigten Variante werden Folie 2 und Vliesstoff 1 mittels Klebstoff laminiert. In diesem Fall kann die Verbindung vollflächig oder auch nur teilflächig erfolgen. Der Klebstoff kann z.B. punktuell oder linienförmig aufgetragen sein.

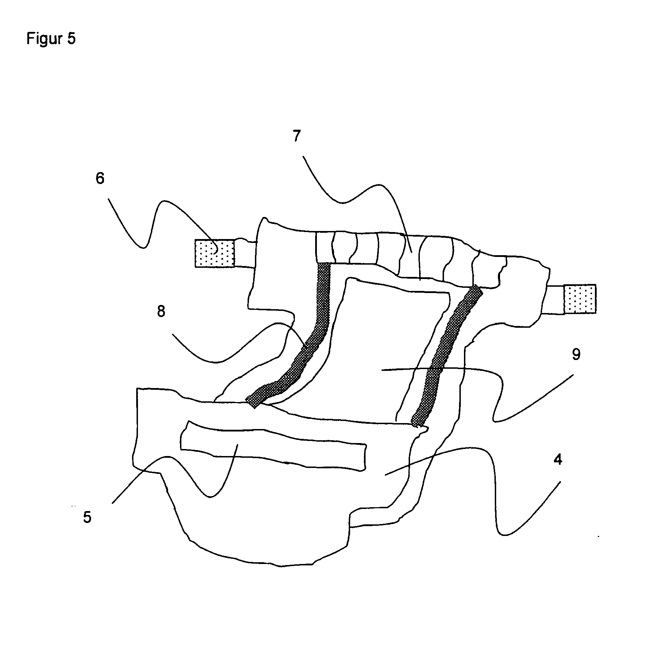

Figur 5 veranschaulicht den Aufbau einer Windel. Die Außenhaut der Windel wird von einem Verbund aus Vliesstoff und Folie, dem Backsheet 4 gebildet. Die Schlaufenkomponente muss zumindest in der Landing Zone 5 vorhanden sein. Entweder wird dort eine erfindungsgemäße Schlaufenkomponente aus Vliesstoff 1 oder aus Vliesstoff 1 und Folie 2 aufgebracht, z.B. aufgeklebt, oder das gesamte Backsheet wird von einem Verbund aus Vliesstoff 1 und Folie 2 gebildet. Eine typische Windel verfügt außerdem über ein elastisches Bündchen 7 in der Taille sowie elastsiche Beinabschlüsse 8. Der Saugkern 9 wird zwischen dem Backsheet 4 und einem Topsheet angeordnet und umfasst in der Regel ein Gemisch aus saugfähigen Faser wie Zellstoffwatte und einem Superabsorber. Superabsorber sind Polymere, die ein Vielfaches ihres Eigengewichtes an Flüssigkeit aufsaugen können.

Beispiele:

### Bestimmung der Scherkraft

| | | |
|---|---|---|
| Hilfsmittel: | Schlaufen - Prüfling | 50 mm * 150 mm |
| | Hakentape | 25,4 mm * 20 mm |
| | Metallplatte | 50 mm * 150 mm |
| | Anrollvorrichtung | 2 kg + 5 kg |
| | Klebeband einseitig (25,4 mm) und zweiseitig (50 mm) | |

Der Schlaufenprüfling wird mit doppelseitigem Klebeband auf die Metallplatte geklebt und mit der Anrollvorrichtung 2 kg ohne Druck 2 mal angerollt. Das Hakentape wird aufs einseitige Klebeband geklebt. Dieses Hakentape wird auf den Schlaufenprüfling mittig der 50 mm schmalen Seite fixiert und 4mal mit der Anrollvorrichtung 5 kg belastet. Der Schlaufenprüfling wird im Reißgerät unten eingespannt, das Hakentape wird im Reißgerät oben eingespannt und bei einer Abzugsgeschwindigkeit von 300 mm/min wird die Scherkraft 180° in N ermittelt.

### Bestimmung der Peelkraft

| | | |
|---|---|---|
| Hilfsmittel: | Schlaufen - Prüfling | 50 mm * 150 mm |
| | Hakentape | 25,4 mm * 20 mm |
| | Gewicht | 500 g zum Hängen |
| | Klebeband einseitig | 25,4 mm breit 100 mm lang |
| | Klemme | 2 Stück, 50 mm Breit |

Das Hakentape wird mittig auf das einseitige Klebeband fixiert. Das Hakentape wird mittig auf den Vliesprüfling fixiert und 3 Sekunden mit 2 Fingern zusammengepresst. Dann wird der Schlaufenprüfling senkrecht auf gehängt und das Hakentape für 5 Sekunden mit 500 g belastet (Scherrichtung). Der Schlaufenprüfling wird unten im Reißgerät senkrecht eingespannt, das nach unten zeigende Hakentape-Ende wird oben im Reißgerät eingespannt und bei einer Abzugsgeschwindigkeit von 300 mm/min wird die Peelkraft in N ermittelt.

Sämtliche Scher- und Peelkräfte werden gemäß der Erfindung entsprechend den obigen Verfahren ermittelt. Die Kräfte werden in N/2,54 cm (N/inch) angegeben. Eine Umrechnung in N/1 cm ist nicht möglich, für andere Abmessungen müssen Bestimmungen an Prüflingen mit diesen Abmessungen erfolgen.

### Beispiel 1

Ein reines wasserstrahlverfestigtes Vlies aus Endlosfilamenten in einer Gewichtsklasse von 35 g/m² wurde als Schlaufenkomponente eines Haken-/Schlaufenverschlusssystems für ein Erwachsenen-Inkontinenzprodukt eingesetzt. Das Vlies, RKW HyJet® 35 g/m², wurde dabei in der Form eines Gürtels über die gesamte Breite des Inkontinenzproduktes eingesetzt und der Verschluss erfolgt mit einem Haken der Fa. Binder; Mikroplast 65445-C. Die für die Prüflinge ermittelten Scherkräfte betragen 65 N, die Peelkraftwerte 45 N.

### Beispiel 2:

Als Landingzone-Material für eine Babywindel wurde ein 25 g/m² wasserstrahlverfestigtes Vlies aus Endlosfilamenten, RKW HyJet® 25 g/m², mit einer Polypropylen-Folie mit einem Gewicht mit 20 g/m² laminiert, so dass das Gesamtcomposit ein Gewicht von 45 g/m² aufwies. Von diesem Laminat wurden Abschnitte in einer Größe von 15 x 4 cm auf den Bauchbereich (Landingzone-Bereich) der Windel appliziert. In diesem Bereich der Windel können dann zum Zwecke des Verschließens der Windel die Hakentapes, beispielsweise die Hakenvariante der Fa. Binder; Mikroplast 25445, verankert werden. Die für die Prüflinge ermittelten Scherkräfte betragen 45 N und die Peelwerte 7 N.

### Bezugszeichenliste

- 1: Vliesstoff
- 2: Folie
- 3: Klebstoff
- 4: Backsheet
- 5: Landing Zone
- 6: Hakentape
- 7: Bündchen
- 8: Beinabschluss
- 9: Saugkern

- B: Bondierungspunkt

## Patentansprüche

1. Verwendung eines wasserstrahlverfestigten Vliesstoffes (1) aus Endlosfilamenten als weibliche Komponente (Schlaufenkomponente) eines Klettverschlusssystems **dadurch gekennzeichnet, dass** in den Vliesstoff (1) keine Öffnungen eingebracht werden und der Vliesstoff (1) vor der Wasserstrahlverfestigung keine Bondierung aufweist.

2. Verwendung gemäß Anspruch 1, **dadurch gekennzeichnet, dass** die Endlosfilamente des wasserstrahlverfestigten Vliesstoffes (1) in definierten Flächen bondiert werden, wobei die Bondierung 1-25 %, vorzugsweise 2 bis 20 % der Gesamtfläche beträgt.

3. Verwendung gemäß Anspruch 1 oder 2, **dadurch gekennzeichnet, dass** der Vliesstoff (1) mit mindestens einer weiteren Lage (2), vorzugsweise einer Folie, verbunden wird, vorzugsweise durch Extrusionsbeschichtung, Kleberlaminierung, Ultraschallverschweißen oder durch Thermolaminierung.

4. Verwendung gemäß Anspruch 3, **dadurch gekennzeichnet, dass** die Schlaufen des Vliesstoffes (1) in das Material der weiteren Lage (2) oder einen Klebstoff (3), mit dem der Vliesstoff (1) und die weitere Lage (2) verbunden werden, auf 1 bis 50 % der Dicke des Vliesstoffes eindringen.

5. Verwendung gemäß mindestens einem der Ansprüche 1 bis 4, **dadurch gekennzeichnet, dass** der Vliesstoff (1) als Schlaufenkomponente zur Fixierung eines absorbierenden Artikels, insbesondere einer Windel oder Einlage, auf diesen aufgebracht wird.

6. Verwendung gemäß Anspruch 3 oder 4, **dadurch gekennzeichnet, dass** der mit der weiteren Lage (2) verbundene Vliesstoff (1) das Backsheet (4) eines Hygienartikels, insbesondere einer Windel, bildet.

## Claims

1. Use of a spunlaced non-woven material (1) of continuous filaments as female component (loop component) of a hook-and-loop fastening system, **characterized in that** no openings are introduced into the non-woven material (1) and the non-woven material (11) does not encompass any bonding prior to the spunlacing.

2. Use according to claim 1, **characterized in that** the continuous filaments of the spunlaced non-woven material (1) are bonded in defined areas, wherein the bonding is 1-25%, preferably 2 to 20% of the total surface.

3. Use according to claim 1 or 2, **characterized in that** the non-woven material (1) is connected to at least one further layer (2), preferably a film, preferably by means of extrusion coating, adhesive lamination, ultrasound welding or by means of thermal lamination.

4. Use according to claim 3, **characterized in that** the loops of the non-woven material (1) penetrate into the material of the further layer (2) or an adhesive (3), with which the non-woven material (1) and the further layer (2) are connected, to 1 to 50% of the thickness of the non-woven material.

5. Use according to a least one of claims 1 to 4, **characterized in that** the non-woven material (1) is applied to an absorbent article, in particular a diaper or pad as loop component for fastening the latter.

6. Use according to claim 3 or 4, **characterized in that** the non-woven material (1), which is connected to the further layer (2) forms the back sheet (4) of a sanitary product, in particular of a diaper.

## Revendications

1. Utilisation d'un non-tissé (1) consolidé par jet d'eau constitué de filaments continus en tant que composant femelle (composant à boucles) d'un système de fermeture auto-grippante, **caractérisée en ce qu'**aucune ouverture n'est formée dans le non-tissé (1) et le non-tissé (1) ne présente pas de liaison avant la consolidation par jet d'eau.

2. Utilisation selon la revendication 1, **caractérisée en ce que** les filaments continus du non-tissé (1) consolidé par jet d'eau sont liés selon des surfaces définies, la liaison représentant 1 à 25 %, de préférence 2 à 20 % de la surface totale.

3. Utilisation selon la revendication 1 ou 2, **caractérisée en ce que** le non-tissé (1) est assemblé avec au moins une autre couche (2), de préférence une feuille, de préférence par revêtement par extrusion, stratification par collage, soudage par ultrasons ou stratification par voie thermique.

4. Utilisation selon la revendication 3, **caractérisée en ce que** les boucles du non-tissé (1) pénètrent dans la matière de l'autre couche (2), ou dans un adhésif (3) servant à assembler le non-tissé (1) et l'autre couche (2), sur 1 à 50 % de l'épaisseur du non-tissé.

5. Utilisation selon au moins l'une des revendications 1 à 4, **caractérisée en ce que** le non-tissé (1) en tant que composant à boucles pour la fixation d'un article absorbant, en particulier d'une couche-culotte ou d'une serviette hygiénique, est appliqué sur celui-ci.

6. Utilisation selon la revendication 3 ou 4, **caractérisée en ce que** le non-tissé (1) assemblé avec l'autre couche (2) forme la feuille de support (4) d'un article d'hygiène, en particulier d'une couche-culotte.
